# FASCICULE DE BREVET EUROPEEN

(11) **EP 1 682 503 B1**
(45) Date de publication et mention de la délivrance du brevet: **11.04.2007**
(21) Numéro de dépôt: 04791553.3
(22) Date de dépôt: 15.10.2004
(51) Int. Cl.: C07D 211/28

(54) **DERIVES DE N-[PHENYL(ALKYLPIPERIDIN-2-YL)METHYL]BENZAMIDE, LEUR PREPARATION ET LEUR APPLICATION EN THERAPEUTIQUE**
DERIVATE VON N-[PHENYL(ALKYLPIPERIDIN-2-YL)METHYL]BENZAMID, VERFAHREN ZU DEREN HERSTELLUNG UND DEREN ANWENDUNG IN THERAPEUTIKA
DERIVATIVES OF N-[PHENYL(ALKYLPIPERIDINE-2-YL)METHYL]BENZAMIDE, PREPARATION METHOD THEREOF AND APPLICATION OF SAME IN THERAPEUTICS

(30) Priorité: 17.10.2003 FR 0312141
(43) Date de publication de la demande: 26.07.2006
(73) Titulaire: Sanofi-Aventis, 75013 Paris (FR)
(72) Inventeur: DARGAZANLI, Gihad, F-94230 Cachan (FR); ESTENNE-BOUHTOU, Geneviève, F-94550 Chevilly-Larue (FR); VERONIQUE, Corinne, F-92160 Antony (FR)
(74) Mandataire: Monain, Patrice
(86) Numéro de dépôt international: PCT/FR2004/002642
(87) Numéro de publication internationale: WO 2005/037782

(56) Documents cités:
- WO-A-99/45011

## Description

Les composés de l'invention répondent à la formule générale (I) 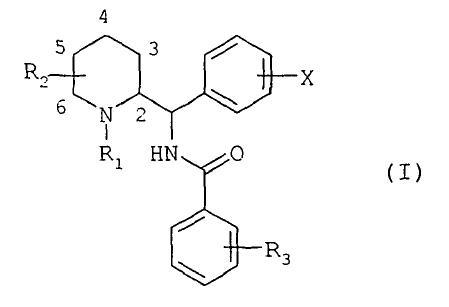 dans laquelle
R₁ représente soit un atome d'hydrogène, soit un groupe (C₁-C₇)alkyle linéaire ou ramifié éventuellement substitué par un ou plusieurs atomes de fluor, soit un groupe (C₃-C₇)-cycloalkyle, soit un groupe (C₃-C₇)cycloalkyl(C₁-C₃)alkyle, soit un groupe phényl(C₁-C₃)alkyle éventuellement substitué par un ou deux groupes méthoxy, soit un groupe (C₂-C₄)alcényle, soit un groupe (C₂-C₄)alcynyle;
R₂ représente soit un groupe (C₁-C₇)alkyle linéaire ou ramifié ou (C₃-C₇)cycloalkyle, soit un groupe (C₃-C₇)cycloalkyl(C₁-C₃)alkyle ;
X représente soit un atome d'hydrogène, soit un ou plusieurs substituants choisis parmi les atomes d'halogène et les groupes trifluorométhyle, (C₁-C₆)alkyle linéaire ou ramifié ou (C₁-C₆) alcoxy ;
R₃ représente soit un atome d'hydrogène, soit un ou plusieurs substituants choisis parmi les atomes d'halogène et les groupes trifluorométhyle, (C₁-C₆)alkyle linéaire ou ramifié, (C₃-C₇)cycloalkyle, (C₁-C₆) alcoxy, phényle, cyano, acétyle, benzoyle, (C₁-C₆)thioalkyle, (C₁-C₆)alkylsulfonyle, carboxy ou (C₁-C₆) alcoxycarbonyl, soit un groupe de formule générale NR₄R₅ ou SO₂NR₄R₅ ou CONR₄R₅ dans lesquelles R₄ et R₅ représentent chacun, indépendamment l'un de l'autre, un atome d'hydrogène ou un groupe (C₁-C₆)alkyle linéaire ou ramifié ou (C₃-C₇)cycloalkyle, ou R₄ et R₅ forment, avec l'atome d'azote qui les porte, un cycle pyrrolidine, pipéridine ou morpholine.

Les composés de formule (I) possèdent deux ou trois centres asymétriques selon que R₂ est en position 2 ou 3, 4, 5 et 6. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Plus particulièrement, les composés de formule (I) peuvent exister sous forme d'énantiomères ou de diastéréoisomères thréo ( (1*S*, 2*S*) et (1*R*, 2*R*)) ou érythro ((1*S*, 2*R*) et (1*R*, 2*S*)) avec une stéréochimie des substituants de la pipéridine cis ou trans ou en mélange de tels isomères.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.
Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Les composés de l'invention présentent une activité particulière comme inhibiteurs spécifiques des transporteurs de la glycine glyt1 et / ou glyt2.

Les composés de formule générale (I) dans laquelle R₁ est différent d'un atome d'hydrogène peuvent être préparés par un procédé illustré par le schéma 1 qui suit.

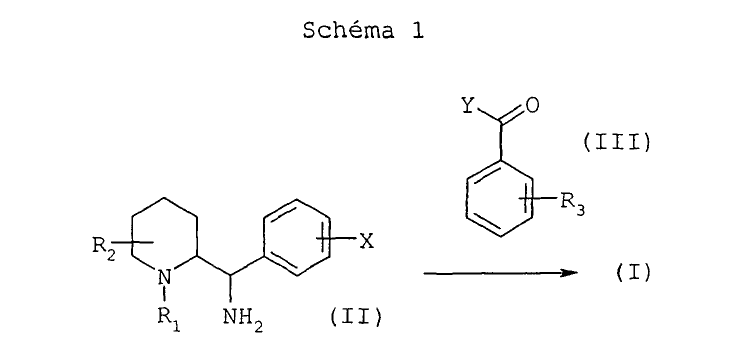

On effectue un couplage d'une diamine de formule générale (II), dans laquelle R₁, R₂ et X sont tels que définis ci-dessus (avec R₁ différent d'un atome d'hydrogène) avec un acide activé ou un chlorure d'acide de formule générale (III) dans laquelle Y représente un groupe OH activé ou un atome de chlore et R₃ est tel que défini ci-dessus, en utilisant les méthodes connues de l'homme du métier.

La diamine de formule générale (II) avec R₂ en position 3, 4, 5 ou 6 peut être préparée par un procédé illustré par le schéma 2 qui suit.
Selon une première voie, on réalise l'alpha-lithiation de la pipéridine de formule générale (IV), dans laquelle R₂ est tel que défini ci-dessus et Boc représente un groupe 1,1-diméthyléthoxycarbonyle, par le *sec*butyllithium en présence de TMEDA (*N,N,N',N'*-tétraméthyléthylènediamine) dans un solvant éthéré tel que l'éther diéthylique à -78°C, pour faire réagir la lithioamine formée *in situ* sur le dérivé de benzaldéhyde de formule générale (VI), dans laquelle X est tel que défini ci-dessus. On obtient ainsi un mélange d'alcool de formule générale (VIII) et de carbamate cyclique de formule générale (IX).
Ces composés peuvent être également obtenus selon une seconde voie de la manière suivante : l'aldéhyde de formule générale (V) est soit préparé selon des méthodes décrites dans la littérature, soit préparé à partir de la pipéridine de formule générale (IV) après lithiation et condensation sur le diméthylformaldéhyde dans les conditions précédemment décrites. On le fait ensuite réagir avec le dérivé de formule générale (VII), dans laquelle X est tel que défini ci-dessus et M représente un métal tel que le lithium, dans un solvant éthéré tel que l'éther diéthylique, entre -30°C et la température ambiante pour donner les composés de formules générales (VIII) et (IX). L'alcool de formule générale (VIII) de configuration threo / erythro est transformé en alcool de formule générale (XI) de configuration thréo en deux étapes de la manière suivante : on oxyde l'alcool en cétone de formule générale (X) par un agent oxydant tel que le chlorochromate de pyridinium dans un solvant chloré tel que le dichlorométhane à température ambiante, puis on réduit de manière diastéréosélective la cétone en alcool de configuration thréo de formule générale (XI) par un agent réducteur tel que le K-Selectride® ou le L-Selectride® (tri-sec-butylborohydrure de potassium ou de lithium), dans un solvant éthéré tel que le tétrahydrofurane, entre -78°C et la température ambiante.
Le carbamate de formule générale (XI) de configuration threo peut ensuite être réduit en N-méthylaminoalcool thréo de formule générale (XII) par action d'un hydrure mixte tel que l'hydrure double d'aluminium et de lithium, dans un solvant éthéré tel que le tétrahydrofurane, entre la température ambiante et la température de reflux.
Dans les mêmes conditions de réduction le carbamate cyclique thréo de formule générale (IX) conduit également au dérivé N-méthylaminoalcool thréo de formule générale (XII).
Le mélange de carbamate cyclique thréo / érythro de formule générale (IX) conduit au mélange de dérivés thréo / érythro de formule générale (XII) que l'on peut purifier et séparer par chromatographie sur gel de silice pour donner le composé thréo pur et le composé érythro pur.

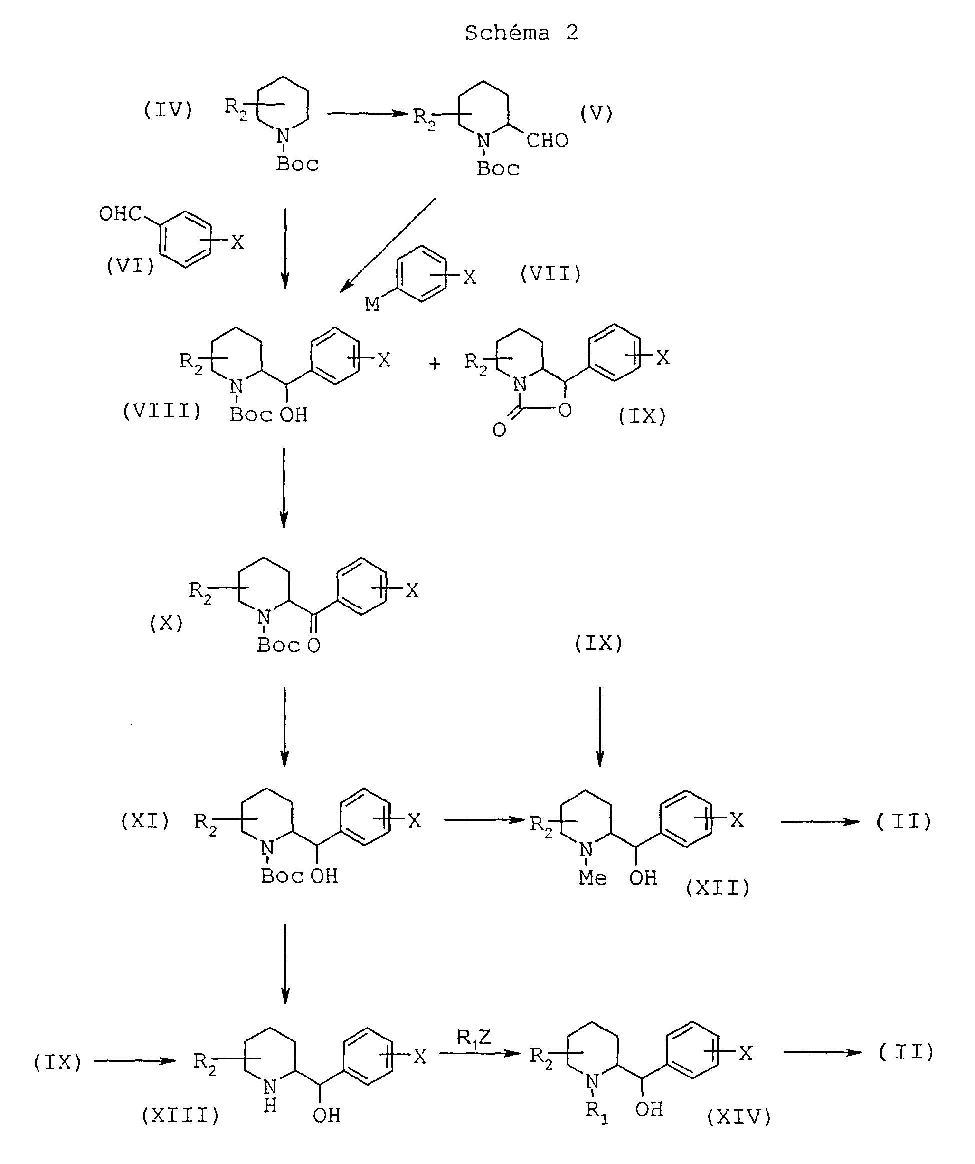

On transforme ensuite l'alcool thréo de formule générale (XII) en intermédiaire thréo de formule générale (II) où R₁ représente un groupe méthyle, en deux étapes: on transforme d'abord la fonction alcool en groupe nucléofuge, par exemple un groupe méthanesulfonate, par action du chlorure de méthylsulfonyle, dans un solvant chloré tel que le dichlorométhane, et en présence d'une base telle que la triéthylamine, entre 0°C et la température ambiante, puis on fait réagir le groupe nucléofuge avec de l'ammoniac liquéfié à -50°C, dans un alcool tel que l'éthanol, dans un milieu clos tel qu'un autoclave, entre -50°C et la température ambiante.
On peut également déprotéger le carbamate de formule générale (XI) de configuration thréo au moyen d'une base forte telle que la potasse aqueuse, dans un alcool tel que le méthanol pour obtenir l'aminoalcool thréo de formule générale (XIII). Dans les mêmes conditions d'hydrolyse le carbamate cyclique de formule générale (IX) conduit à l'aminoalcool de formule générale (XIII).
On procède ensuite à une *N*-alkylation au moyen d'un dérivé halogéné de formule R₁Z, dans laquelle R₁ est tel que défini ci-dessus, mais différent d'un atome d'hydrogène, et Z représente un atome d'halogène, en présence d'une base telle que le carbonate de potassium, dans un solvant polaire tel que le *N,N*-diméthylformamide, entre la température ambiante et 100°C pour conduire au dérivé alkylé de formule générale (XIV). On transforme ensuite le dérivé alkylé de formule générale (XIV)en intermédiaire de formule générale (II) comme décrit à propos de l'alcool de formule générale (XII).
On peut procéder de la même manière que ci-dessus avec les dérivés de formule générale (IX) érythro pour obtenir les composés de formule générale (I) érythro.

La diamine de formule générale (II) avec R₂ en position 2 peut être préparée par un procédé illustré par le schéma 3 qui suit.

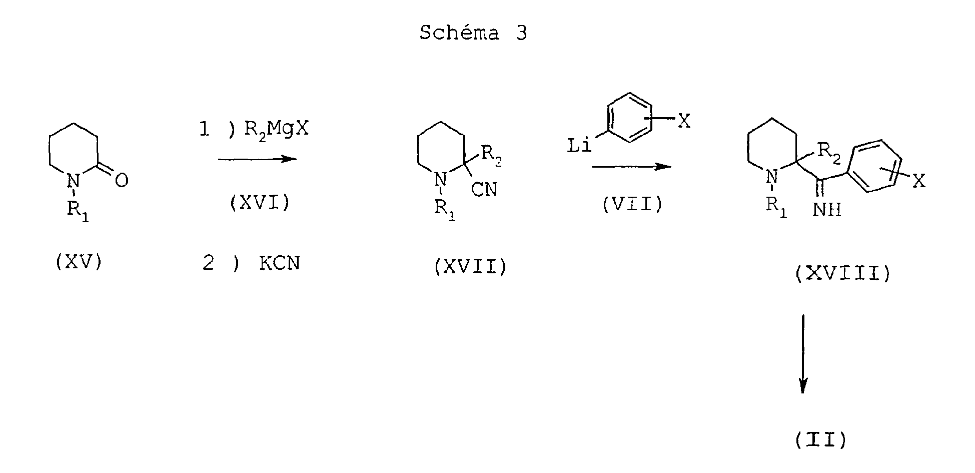

On fait réagir une pipéridinone de formule générale (XV), dans laquelle R₁ est tel que défini ci-dessus, avec un organométallique de formule générale (XVI), à reflux dans le tétrahydrofurane et on traite le milieu réactionnel par une solution de cyanure de potassium pour conduire à l'amino-nitrile de formule générale (XVII). On fait réagir ensuite ce dernier avec un dérivé lithié de formule générale (VII), dans laquelle X est tel que défini ci-dessus, dans un solvant éthéré tel que l'éther diéthylique ou le tétrahydrofurane, entre -90°C et -30°C ; on obtient une imine intermédiaire de formule générale (XVIII) que l'on réduit en amine primaire thréo de formule générale (II) par un agent réducteur tel que le borohydrure de sodium, dans un solvant protique tel que le méthanol, entre 0°C et la température ambiante.

Les composés de formule générale (I) dans laquelle R₁ représente un atome d'hydrogène peuvent être préparés à partir d'un composé de générale (I) dans laquelle R₁ représente :
- soit un groupe phénylméthyle éventuellement substitué, en déprotégeant l'azote du cycle pipéridine, par exemple par un agent oxydant ou par un acide de Lewis tel que le tribromure de bore, ou par hydrogénolyse,
- soit un groupe alcényle, de préférence allyle, en déprotégeant l'azote du cycle pipéridine, par exemple par un complexe du Pd° pour obtenir un composé de formule générale (I) dans laquelle R₁ représente un atome d'hydrogène.

La pipéridinone de formule générale (XV) est disponible dans le commerce.

Par ailleurs les composés chiraux de formule générale (I) peuvent être obtenus par séparation des composés racémiques par chromatographie liquide à haute performance (CLHP) sur colonne chirale, ou par dédoublement de l'amine racémique de formule générale (II) par utilisation d'un acide chiral, tel que l'acide tartrique, l'acide camphorsulfonique, l'acide dibenzoyltartrique, la N-acétylleucine, par la recristallisation fractionnée et préférentielle d'un sel diastéréoisomérique dans un solvant de type alcool.

Les pipéridines de formules générales (IV) sont préparées par protection, par un groupe Boc par exemple, de l'azote des pipéridines correspondantes, disponibles dans le commerce ou décrites dans la littérature, selon des méthodes connues de l'homme du métier. La méthode de formation de la lithiopipéridine à partir de la pipéridine de formule générale (IV) et sa réaction sur le benzaldéhyde de formule générale (VI) est analogue à celle décrite dans *J.O.C.,* **55,** (1990), 2578-2580. Le composé phényllithié de formule générale (VII) où X représente un atome d'hydrogène est disponible dans le commerce. Ses dérivés substitués peuvent être préparés selon une méthode analogue à celle décrite dans *Tetrahedron Lett.,* **57**, 33, (1996), 5905-5908. Les aldéhydes de formule générale (V) où R₂ représente un groupe méthyle dans les positions 2, 4, 5 et 6 sont décrits dans *J. O. C.,* **58,** (1993), 1109-117 et *J. Chem.Soc.,Perkin Trans.1,* (2002), 1438-1443. Les dérivés halogénés de formule R₁Z sont disponibles dans le commerce. Certains acides et chlorures d'acides de formule générale (III) sont disponibles dans le commerce ou, lorsqu'ils sont nouveaux, ils peuvent être obtenus selon des méthodes analogues à celles décrites dans les brevets EP-0556672, US-3801636, et dans *J. Chem. Soc*., (1927), 25, *Chem. Pharm. Bull. ,* (1992), 1789-1792, *Aust. J. Chem.,* (1984), 1938-1950 et *J. O. C.,* (1980), 527.

Les exemples qui vont suivre illustrent la préparation de quelques composés de l'invention. Les microanalyses élémentaires, les spectres I.R. et R.M.N. et la CLHP sur colonne chirale confirment les structures et les puretés énantiométriques des composés obtenus.
Les numéros indiqués entre parenthèses dans les titres des exemples correspondent à ceux de la 1ère colonne du tableau donné plus loin.
Dans les noms des composés, le tiret "-" fait partie du mot, et le tiret "-" ne sert que pour la coupure en fin de ligne ; il est à supprimer en l'absence de coupure, et ne doit être remplacé ni par un tiret normal ni par un espace.

### Exemple 1 (Composé N°1).

### Chlorhydrate de cis-thréo-2-chloro-N-[(1,6-diméthylpipéridin-2-yl) (phényl)méthyl]-3-(trifluorométhyl)benzamide 1:1.

### 1.1. Cis [2-hydroxy(phényl)méthyl-6-méthyl]pipéridine-1-carboxylate de 1,1-diméthyléthyle.

Dans un ballon de 50 ml, sous atmosphère d'azote, on introduit 1 g (4,4 mmoles) de cis 2-formyl-6-méthylpipéridine-1-carboxylate de 1,1-diméthyléthyle dans 15 ml de tétrahydrofurane anhydre, on refroidit le milieu à -78°C, on ajoute, goutte à goutte, 4,4 ml (4,4 mmoles) d'une solution 1M de bromure de phénylmagnésium dans le tétrahydrofurane et on laisse le mélange revenir à -50°C sous agitation pendant 2 h.

Après hydrolyse avec une solution aqueuse saturée de chlorure d'ammonium on sépare la phase aqueuse et on l'extrait avec de l'acétate d'éthyle. On sèche les phases organiques réunies sur sulfate de sodium, on filtre, on concentre sous pression réduite et on purifie le résidu par chromatographie sur colonne de gel de silice en éluant avec un mélange d'acétate d'éthyle et de cyclohexane.
On obtient 1,15 g d'alcool sous forme d'un mélange de diastéréoisomère thréo / érythro.

### 1.2. Cis 2-méthyl-6-(phénylcarbonyl)pipéridine-1-carboxylate de 1,1-diméthyléthyle.

Dans un ballon de 100 ml, on introduit successivement 0,12 g (1,5 mmoles) d'acétate de sodium en suspension dans 20 ml de dichlorométhane et 1,2 g (5,5 mmoles) de chlorochromate de pyridinium, puis on ajoute une solution de 1,15 g (3,76 mmoles) de cis [2-hydroxy(phényl)méthyl-6-méthyl]pipéridine-1-carboxylate de 1,1-diméthyléthyle dans 20 ml de dichlorométhane. Le mélange devient noir rapidement et on le laisse sous agitation à température ambiante pendant 4 h.
On ajoute 30 ml d'éther éthylique, on filtre, on rince, on concentre sous pression réduite et on purifie le résidu par chromatographie sur colonne de gel de silice en éluant avec un mélange d'acétate d'éthyle et de cyclohexane.
On obtient 0,46 g de cétone sous forme de solide blanc. Point de fusion : 92-93°C.

### 1.3 Cis-thréo-[2-hydroxy(phényl)méthyl-6-méthyl]pipéridine-1-carboxylate de 1,1-diméthylèthyle.

Dans un ballon de 100 ml, sous atmosphère d'argon, on introduit 0,46 g (1,5 mmoles) de cis 2-méthyl-6-(phénylcarbonyl)pipéridine-1-carboxylate de 1,1-diméthyléthyle dans 40 ml de tétrahydrofurane anhydre, on refroidit la solution à -78°C, on ajoute, goutte à goutte, 4,6 ml (4,6 mmoles) d'une solution 1M de L-Selectride® (tri-sec-butylborohydrure de lithium) dans le tétrahydrofurane, et on agite le mélange à -78°C pendant 5 h.
On l'hydrolyse à froid lentement avec 3,2 ml d'eau et 3,2 ml d'une solution aqueuse à 35% de peroxyde d'hydrogène, et on laisse le mélange revenir à température ambiante en l'agitant pendant 2 h.
On le dilue avec de l'eau et de l'acétate d'éthyle, on sépare les phases et on extrait la phase aqueuse avec de l'acétate d'éthyle. Après lavage des phases organiques réunies, séchage sur sulfate de sodium, filtration et évaporation on purifie le résidu par chromatographie sur colonne de gel de silice en éluant avec un mélange d'acétate d'éthyle et de cyclohexane.
On obtient 0,38 g d'isomère thréo-cis sous d'une huile incolore.

1.4. Cis-thréo-(1,6-diméthylpipéridin-2-yl)phénylméthanol. Dans un bicol de 25 ml, sous atmosphère d'azote, on introduit 0,24 g (6,3 mmoles) d'hydrure double d'aluminium et de lithium dans 7 ml de tétrahydrofurane anhydre, on chauffe le mélange au reflux, on ajoute 0,38 g (1,2 mmole) d'une solution de cis-thréo-[2-hydroxy(phényl)méthyl-6-méthyllpipéridine-1-carboxylate de 1,1-diméthyléthyle dans 3 ml de tétrahydrofurane et on maintient le mélange au reflux pendant 3,5 h.
On le refroidit, on l'hydrolyse lentement avec une solution 0,1M de tartrate double de potassium et de sodium et on laisse le mélange sous agitation pendant une nuit.
On le filtre, on rince le précipité avec du tétrahydrofurane, on concentre le filtrat sous pression réduite et on purifie le résidu par chromatographie sur colonne de gel de silice en éluant avec un mélange de dichlorométhane et de méthanol.
On obtient 0,11 g d'un produit huileux incolore.

### 1.5. Cis-thréo-(1,6-diméthylpipéridinyl-2-yl)phénylméthanamine.

Dans un ballon de 25 ml, sous atmosphère d'azote, on introduit 0,11 g (0, 52 mmoles) de cis-thréo-(1,6-diméthylpipéridin-2-yl)phénylméthanol et 0,11 ml (0,78 mmole) de triéthylamine dans 7 ml de dichlorométhane anhydre, on refroidit le milieu à 0°C, on ajoute 0,06 ml (0,78 mmole) de chlorure de méthanesulfonyle, on laisse le mélange revenir lentement à température ambiante pendant 2 h et on le concentre sous pression réduite.
Dans un autoclave muni d'une agitation magnétique et refroidi à -50°C on introduit de l'ammoniac liquéfié, on ajoute une solution du méthanesulfonate brut précédemment préparé en solution dans 30 ml d'éthanol absolu, on ferme l'autoclave et on maintient l'agitation pendant 48 h.
On transvase le mélange dans un ballon, on concentre à sec, on dilue le résidu avec de l'eau et du dichlorométhane, on sépare les phases et on extrait la phase aqueuse avec du dichlorométhane. Après lavage des phases organiques réunies, séchage sur sulfate de sodium, filtration et évaporation on isole 0,1 g d'amine sous forme de composé huileux que l'on utilise tel quel dans l'étape suivante.

### 1.6. Chlorhydrate de cis-thréo-2-chloro-N-[(1,6-diméthylpipéridin-2-yl)(phényl)méthyl]-3-(trifluorométhyl)-benzamide 1:1.

Dans un ballon de 25 ml on introduit successivement 0,13 g (0,58 mmole) d'acide 2-chloro-3-trifluorométhanebenzoïque, 0,11 g (0,59 mmole) de chlorhydrate de 1-[3-(diméthylamino)propyl]-3-éthylcarbodiimide, 0,03 g (0,24 mmole) de diméthylaminopyrine en solution dans 4 ml de dichlorométhane et on ajoute 0,10 g (0,48 mmole) de cis-thréo-(1,6-diméthylpipéridinyl-2-yl)phénylméthanamine en solution dans 1 ml de dichlorométhane et on laisse le mélange sous agitation pendant 5 h.
On le traite avec de l'eau, on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol.
On obtient 0,18 g de produit huileux qu'on isole sous forme de chlorhydrate à partir d'une solution 0,1N d'acide chlorhydrique dans le propan-2-ol.
On isole finalement 0,12 g de chlorhydrate sous forme de solide blanc.
Point de fusion : 208-209°C.

### Exemple 2 (Composé N°9).

### Chlorhydrate de cis-thréo-2-chloro-N-[(1,4-diméthylpiperidinyl-2-yl) (phényl)méthyl] -3-(trifluorométhyl)benzamide 1:1

### 2.1. Cis-7-méthyl-1-phénylhexahydro[1,3]oxazolo[3,4-a]pyridin-3-one.

Dans un ballon de 1 l, sous atmosphère d'argon, on introduit 14,2 g (71,2 mmoles) de 4-méthylpipéridine-1-carboxylate de 1,1-diméthyléthyle en solution dans 130 ml d'éther diéthylique anhydre et on refroidit le milieu à - 70°C. On ajoute 14 ml (92,5 mmoles) de TMEDA (*N,N,N'*,*N'*-tétraméthyléthylènediamine) puis 70 ml (92, 5 mmoles) d'une solution 1,3M de sec-butyllithium dans le cyclohexane, et on laisse revenir à -30°C sous agitation pendant 0,5 heure.
On ajoute ensuite une solution de 11,33 ml (106,8 mmoles) de benzaldéhyde (préalablement distillé) dans 40 ml d'éther diéthylique anhydre et on laisse sous agitation le mélange revenir à température ambiante pendant 12 h.
Après hydrolyse avec de l'eau, on sépare la phase aqueuse et on l'extrait avec de l'acétate d'éthyle. On sèche les phases organiques réunies sur sulfate de sodium, on filtre, on concentre sous pression réduite et on purifie le résidu par chromatographie sur colonne de gel de silice en éluant avec un mélange de dichlorométhane et de méthanol.
On obtient 3,3 g d'huile incolore d'un mélange d'isomères cis thréo/érytrho.

### 2.2. Cis-thréo-(1,4-diméthylpipéridin-2-yl)phénylméthanol.

Dans un bicol de 500 ml, sous atmosphère d'azote, on introduit 2,71 g (71,5 mmoles) d'hydrure double d'aluminium et de lithium dans 120 ml de tétrahydrofurane anhydre, on chauffe le mélange au reflux, on ajoute 3,33 g (14,3 mmoles) d'une solution de cis 7-méthyl-1-phénylhexahydro[1,3]oxazolo[3,4-a]pyridin-3-one dans 40 ml de tétrahydrofurane et on maintient le mélange au reflux pendant 5,5 h.
On le refroidit, on l'hydrolyse lentement avec une solution 0,1M de tartrate double de potassium et de sodium et on laisse le mélange sous agitation pendant une nuit.
On le filtre, on rinçe le précipité avec du tétrahydrofurane, on concentre le filtrat sous pression réduite et on purifie le résidu par chromatographie sur colonne de gel de silice en éluant avec un mélange de dichlorométhane et de méthanol.
On obtient 0,95 g d'un produit sous forme d'huile incolore.

### 2.3. Cis-thréo-(1,4-diméthylpipéridinyl-2-yl)phénylméthanamine.

Dans un ballon de 10 ml, sous atmosphère d'azote, on introduit 0,95 g (4,3 mmoles) de cis-thréo-(1,4-diméthylpipéridin-2-yl)phénylméthanol et 0,9 ml (6,5 mmoles) de triéthylamine dans 40 ml de dichlorométhane anhydre, on refroidit le milieu à 0°C, on ajoute 0,5 ml (6,5 mmoles) de chlorure de méthanesulfonyle, on laisse le mélange revenir lentement à température ambiante pendant 2 h et on le concentre sous pression réduite.
Dans un autoclave muni d'une agitation magnétique et refroidi à -50°C on introduit de l'ammoniac liquéfié, on ajoute une solution du méthanesulfonate brut précédemment préparé en solution dans 30 ml d'éthanol absolu, on ferme l'autoclave et on maintient l'agitation pendant 48 h.
On transvase le mélange dans un ballon, on concentre à sec, on dilue le résidu avec de l'eau et du dichlorométhane, on sépare les phases et on extrait la phase aqueuse avec du dichlorométhane. Après lavage des phases organiques réunies, séchage sur sulfate de sodium, filtration et évaporation, on isole 0,8 g d'amine sous forme de composé huileux que l'on utilise tel quel dans l'étape suivante.

### 2.4. Chlorhydrate de cis-thréo-2-chloro-N-[(1,4-diméthylpipéridin-2-yl)(phényl)méthyl]-3-(trifluorométhyl)-benzamide 1:1

Dans un ballon de 50 ml on introduit successivement 0,98 g (4,38 mmoles) d'acide 2-chloro-3-trifluorométhanebenzoïque, 0,85 g (4,46 mmoles) de chlorhydrate de 1-[3-(diméthylamino)propyl]-3-éthylcarbodiimide, 0,22 g (1,83 mmole) de diméthylaminopyrine en solution dans 20 ml de dichlorométhane et on ajoute 0,8 g (3,66 mmoles) de cis-thréo-(1,4-diméthylpipéridin-2-yl)phénylméthanamine en solution dans 4 ml de dichlorométhane et on laisse le mélange sous agitation pendant 12 h.
On le traite avec de l'eau, on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol.
On obtient 0,32 g de produit huileux qu'on isole sous forme de chlorhydrate à partir d'une solution 0,1N d'acide chlorhydrique dans le propan-2-ol.
On isole finalement 0,28 g de chlorhydrate sous forme de solide blanc.
Point de fusion : 209-211°C.

### Exemple 3 (Composé N°5) .

### Chlorhydrate de trans-thréo-2-chloro-N-[(1,5-diméthylpiperidinyl-2-yl)(4-fluorophényl)méthyl]-3-(trifluorométhyl)benzamide 1:1.

En procédant de la même manière qu'à l'exemple 2 et en remplaçant le 4-méthylpipéridine-1-carboxylate de 1,1-diméthyléthyle par le 5-méthylpipéridine-1-carboxylate de 1,1-diméthyléthyle et le benzaldéhyde par le 4-fluorobenzaldéhyde, on obtient un mélange d'alcool et d'isoxazolidone correspondants. La réduction de l'isoxazolidone obtenue par de l'hydrure double d'aluminium et de lithium donne le composé aminoalcool thréo trans qui est engagé selon les méthodes décrites aux étapes 2.3 et 2.4 de l'exemple 2 en utilisant d'acide 2-chloro-3-trifluorométhanebenzoïque.
Point de fusion : 220-222°C.

### Exemple 4 (Composé N°10).

### Chlorhydrate de thréo 2-chloro-N-[(1,2-diméthylpipéridin-2-yl)(phényl)méthyl]-3-(trifluorométhyl)benzamide 1:1.

### 4.1. 1,2-Diméthylpipéridine-2-carbonitrile.

Dans un tricol de 500 ml équipé d'un réfrigérant et d'une agitation magnétique on introduit, sous atmosphère d'azote, 22 ml (31 mmoles) d'une solution de bromure de méthyl-magnésium 1,4 M dans le tétrahydrofurane puis une solution de 5 g (44,2 mmoles) de 1-méthylpipéridin-2-one dans 20 ml de tétrahydrofurane, et on chauffe le mélange sous agitation à reflux pendant 2 h.
On laisse refroidir le mélange, on ajoute 50 ml d'une solution 2 N d'acide chlorhydrique et on l'extrait avec de l'acétate d'éthyle. La phase aqueuse est ensuite ajustée à pH 6 à l'aide de bicarbonate de sodium et on ajoute 2,9 g (2,8 mmoles) de cyanure de potassium. On laisse ensuite sous agitation à 25 °C pendant 12 h.
On ajoute une solution à 10% de bicarbonate de sodium et on extrait à l'acétate d'éthyle. Après lavage des phases organiques réunies, séchage sur sulfate de sodium, filtration et évaporation on obtient 3 g de produit sous forme de composé huileux que l'on utilise tel quel dans l'étape suivante

### 4.2. 1-(1,2-dimëthylpipéridin-2-yl)-1-phénylméthanamine.

Dans un ballon de 250 ml muni d'une agitation magnétique et sous atmosphère d'argon, on prépare à -78°C une solution de phényllithium à partir de 6,8 g (43,4 mmoles) de bromobenzène dans 50 ml de tétrahydrofurane et de 17,4 ml de butyllithium (2.5 M dans l'hexane). On introduit, à 78°C, une solution de 3 g (21,71 mmoles) de 1,2-diméthylpipéridine-2-carbonitrile dans 50 ml de tétrahydrofurane et on laisse sous agitation le mélange (solution jaune) revenir à température ambiante pendant 1 h. On ajoute de l'eau et on extrait avec de l'acétate d'éthyle. On sèche les phases organiques réunies sur sulfate de sodium, on filtre et on concentre l'imine sous pression réduite.
On reprend le résidu dans un ballon de 250 ml avec 50 ml de méthanol. On refroidit le mélange à 0°C et on ajoute lentement 4 g (108 mmoles) de borohydrure de sodium. On poursuit l'agitation en laissant la température du mélange revenir à température ambiante pendant 1 h. On concentre le mélange sous pression réduite et on le reprend avec de l'eau et de l'acétate d'éthyle. On sépare les phases et on extrait la phase aqueuse avec de l'acétate d'éthyle. Après lavage des phases organiques réunies, séchage sur sulfate de sodium, filtration et évaporation on obtient 3,2 g de produit sous sous forme de composé huileux que l'on utilise tel quel dans l'étape suivante.

### 4.3 Chlorhydrate de thréo 2-chloro-N-[(1,2-diméthylpipé ridin-2-yl)(phényl)méthyl]-3-(trifluorométhyl)benzamide 1:1.

Dans un ballon de 100 ml on introduit successivement, dans 20 ml de dichlorométhane, 0,41 g (1,8 mmole) de 1-(1,2-diméthylpipéridin-2-yl)-1-phénylméthanamine, 0,3 ml (2,25 mmoles) de triéthylamine , 0,54 g (2,25 mmoles) de chlorure d'acide 2-chloro-3-(trifluorométhyl)benzoïque et on agite le mélange à température ambiante pendant 1 h.
On le traite avec de l'eau et on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol.
On obtient 0,36 g de produit huileux.
On le transforme en chlorhydrate à partir d'une solution 0,1N d'acide chlorhydrique dans le propan-2-ol.
On isole finalement 0,14 g de chlorhydrate sous forme de solide blanc.
Point de fusion : 239-241°C.

Le tableau qui suit illustre les structures chimiques et les propriétés physiques de quelques composés de l'invention.
Dans la colonne "Sel", "HCl" désigne un chlorhydrate.

**Tableau**

| 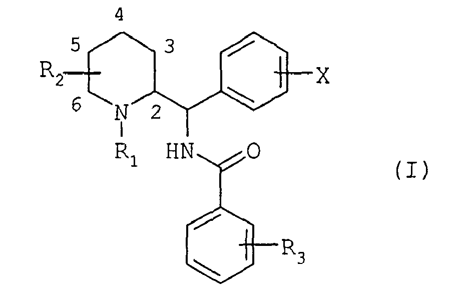 | | | | | | | |
|---|---|---|---|---|---|---|---|
| N° | R₁ | X | R₂ | R₃ | Sel | F (°C) | Stéréochimie |
| 1 | CH₃ | H | Cis 6-CH₃ | 2-Cl, 3-CF₃ | HCl | 208-209 | Thréo |
| 2 | CH₃ | H | Trans 6-CH₃ | 2-Cl, 3-CF₃ | HCl | 128-129 | Thréo |
| 3 | CH₃ | H | Trans 6-CH₂-C6H11 | 2-Cl, 3-CF₃ | HCl | 127-129 | Thréo |
| 4 | CH₃ | H | Trans 6-CH₃ | 3,5-Cl, 4-NH₂ | HCl | 270-271 | Thréo |
| 5 | CH₃ | 4-F | Trans 5-CH₃ | 2-Cl, 3-CF₃ | HCl | 220-222 | Thréo |
| 6 | CH₃ | 4-F | Trans 5-CH₃ | 3-Cl, 4-NH₂ | HCl | 169-171 | Thréo |
| 7 | CH₃ | H | Cis 5-CH₃ | 2-CH₃, 3-CF₃ | HCl | 131-133 | Thréo |
| 8 | CH₃ | 4-F | Cis 6-CH₃ | 3,5-C1, 4-NH₂ | HCl | 254-256 | Thréo |
| 9 | CH₃ | H | Cis 4-CH₃ | 2-Cl, 3-CF₃ | HCl | 209-211 | Thréo |
| 10 | CH₃ | H | 2-CH₃ | 2-Cl, 3-CF₃ | HCl | 239-241 | Thréo |
| 11 | CH₃ | H | 2-CH₃ | 2-Cl, 3-CH₃, 6-F | HCl | 227-229 | Thréo |
| 12 | CH₃ | H | 2-CH₃ | 2-CH₃, 3-CF₃ | HCl | 149-151 | Thréo |
| 13 | CH₃ | H | 2-CH₃ | 2-CH₃, 3-OCH₃ | HCl | 170-172 | Thréo |
| 14 | CH₃ | H | 2-CH₃ | 2-CH₃, 3-Cl | HCl | 177-179 | Thréo |
| 15 | CH₃ | H | 2-CH₃ | 2-Cl, 5-CF₃ | HCl | 213-215 | Thréo |
| 16 | CH₃ | H | 2-CH₃ | 2,6-Cl, 3-CF₃ | HCl | 253-255 | Thréo |
| 17 | CH₂CH:CH₂ | H | 2-CH₃ | 2-Cl, 3-CF₃ | HCl | 244-246 | Thréo |
| 18 | H | H | 2-CH₃ | 2-Cl, 3-CF₃ | HCl | 278-280 | Thréo |

Les composés de l'invention ont été soumis à une série d'essais pharmacologiques qui ont mis en évidence leur intérêt comme substances à activités thérapeutiques.

### Etude du transport de la glycine dans les cellules SK-N-MC exprimant le transporteur humain glyt1 natif.

La capture de [¹⁴C] glycine est étudiée dans les cellules SK-N-MC (cellules neuro-épithéliales humaines) exprimant le transporteur humain glyt1 natif par la mesure de la radioactivité incorporée en présence ou en absence du composé à tester. Les cellules sont cultivées en monocouche pendant 48 h dans des plaques prétraitées à la fibronectine à 0,02%. Le jour de l'expérience, le milieu de culture est éliminé et les cellules sont lavées par un tampon Krebs-HEPES (acide [4-(2-hydroxyéthyl)pipérazine-1-éthanesulfonique) à pH 7,4. Après 10 min de préincubation à 37°C en présence soit de tampon (lot témoin), soit de composé à tester a différentes concentrations ou de 10 mM de glycine (détermination de la capture non spécifique), 10 µM de [¹⁴C]glycine (activité spécifique 112 mCi/mmole) sont ensuite ajoutés. L'incubation se poursuit pendant 10 min à 37°C, et la réaction est arrêtée par 2 lavages avec un tampon Krebs-HEPES à pH 7,4. La radioactivité incorporée par les cellules est alors estimée après ajout de 100 µl de scintillant liquide et agitation pendant 1 h. Le comptage est réalisé sur compteur Microbeta Tri-lux^{™}. L'efficacité du composé est déterminée par la CI₅₀, concentration du composé qui diminue de 50% la capture spécifique de glycine, définie par la différence de radioactivité incorporée par le lot témoin et le lot qui a reçu la glycine à 10 mM.

Les composés de l'invention les plus actifs, dans ce test, ont une CI₅₀ de l'ordre de 0,001 à 10 µM.
les résultats individuels de quelques composés sont les suivants (CI₅₀ en µM) :

| | |
|---|---|
| Composé N°1 | 0,51 |
| Composé N°5 | 0,1 |
| Composé N°9 | 0,09 |
| Composé N°10 | 0,008 |

### Etude ex vivo de l'activité inhibitrice d'un composé sur la capture de la [¹⁴C]glycine dans l'homogénat cortical de souris.

Des doses croissantes du composé à étudier sont administrées par voie orale (préparation par trituration de la molécule à tester dans un mortier dans une solution de Tween/Methocel^{™} à 0,5% dans de l'eau distillée) ou intrapéritonéale (dissolution de la molécule à tester dans du sérum physiologique ou prépation par trituration dans un mortier dans une solution de Tween/methocel à 0,5% dans de l'eau, selon la solubilité de la molécule) sur des souris mâles OF1 Iffa Crédo de 20 à 25 g le jour de l'expérience. Le groupe témoin est traité par le véhicule. Les doses en mg/kg, la voie d'administration et le temps de traitement sont déterminés en fonction de la molécule à étudier.

Après euthanasie par décapitation des animaux à un temps donné après l'administration, le cortex de chaque animal est rapidement prélevé sur glace, pesé et conservé à 4°C ou congelé à -80°C (dans les deux cas les échantillons sont conservés 1 jour maximum). Chaque échantillon est homogénéisé dans un tampon Krebs-HEPES à pH 7,4 à raison de 10 ml/g de tissu. 20 µl de chaque homogénat sont incubés pendant 10 min à température ambiante en présence de 10 mM de L-alanine et de tampon. La capture non spécifique est déterminée par addition de 10 mM de glycine au groupe témoin. La réaction est arrêtée par filtration sous vide et la radioactivité retenue est estimée par scintillation solide par comptage sur compteur Microbeta Tri-lux^{™}.
Un inhibiteur de la capture de la [¹⁴C]glycine diminuera la quantité de radioligand incorporée dans chaque homogénat. L'activité du composé est évaluée par sa DE₅₀, dose qui inhibe 50% de la capture de la [¹⁴C]glycine par rapport au groupe témoin.

Les composés de l'invention les plus puissants, dans ce test, ont une DE₅₀ de 0,1 à 5 mg/kg par voie intrapéritonéale ou par voie orale.

### Etude du transport de la glycine dans l'homogénat de moelle épinière de souris.

La capture de [¹⁴C] glycine par le transporteur glyt2 est étudiée dans l'homogénat de moelle épinière de souris par la mesure de radioactivité incorporée en présence ou en absence de composé à étudier.

Après euthanasie des animaux (souris mâles OF1 Iffa Crédo pesant 20 à 25 g le jour de l'expérience), la moelle épinière de chaque animal est rapidement prélevée, pesée et conservée sur glace. Les échantillons sont homogénéisés dans un tampon Krebs-HEPES (acide [4-(2-hydroxyéthyl)pipérazine-1-éthanesulfonique), pH 7,4, à raison de 25 ml/g de tissu.
50 µl d'homogénat sont pré-incubés pendant 10 min à 25°C en présence de tampon Krebs-HEPES , pH 7,4 et de composé à étudier à différentes concentrations, ou de 10 mM de glycine pour déterminer la capture non spécifique. La [¹⁴C]glycine (activité spécifique = 112mCi/mmole) est ensuite ajoutée pendant 10 min à 25°C à la concentration finale de 10 µM. La réaction est arrêtée par filtration sous vide et la radioactivité est estimée par scintillation solide par comptage sur un compteur Microbeta Tri-lux^{™}. L'efficacité du composé est déterminée par la concentration CI₅₀ capable de diminuer de 50% la capture spécifique de glycine, définie par la différence de radioactivité incorporée par le lot témoin et le lot qui a reçu la glycine 10 mM.

Les composés de l'invention les plus actifs dans ce test, ont une CI₅₀ de l'ordre de 0,001 à 10 µM.
les résultats individuels de quelques composés sont les suivants (CI₅₀ en µM) :

| | |
|---|---|
| Composé N°1 | 0,12 |
| Composé N° 9 | 0,07 |

### Etude ex vivo de l'activité inhibitrice d'un composé sur la capture de la [¹⁴C] glycine dans l'homogénat spinal de souris.

Des doses croissantes du composé à étudier sont aministrées par voie orale (préparation par trituration du composé à tester dans un mortier, dans une solution de Tween/Methocel^{™} à 0,5% dans de l'eau distillée) ou intrapéritonéale (composé à tester dissous dans du sérum physiologique, ou trituré dans un mortier, dans une solution de Tween/Methocel^{™} à 0,5% dans de l'eau distillée) à des souris mâles OF1 Iffa Crédo de 20 à 25 g le jour de l'expérience. Le groupe témoin est traité par le véhicule. Les doses en mg/kg, la voie d'administration, le temps de traitement ainsi que le temps d'euthanasie sont déterminés en fonction du composé à étudier.
Après euthanasie par décapitation des animaux à un temps donné après l'administration, les moelles sont prélevées rapidement, pesées et introduites dans des fioles à scintillation en verre, conservées dans de la glace pilée
ou congelées à -80°C (dans les deux cas les échantillons sont conservés 1 jour maximum). Chaque échantillon est homogénéisé dans un tampon Krebs-HEPES à pH 7,4, à raison de 25 ml/g de tissu. 50 µl de chaque homogénat sont incubés pendant 10 min à température ambiante en présence de tampon.
La capture non spécifique est déterminée par addition de 10 mM de glycine au groupe témoin.
La réaction est arrêtée par filtration sous vide et la radioactivité est estimée par scintillation solide par comptage sur un compteur Microbeta Tri-lux^{™}.
Un inhibiteur de la capture de la [¹⁴C]glycine diminuera la quantité de radioligand incorporée dans chaque homogénat. L'activité du composé est évaluée par sa DE₅₀, dose efficace qui inhibe 50% de la capture de la [¹⁴C]glycine par rapport au groupe témoin.

Les composés de l'invention les plus actifs ont, dans ce test, une DE₅₀ de 1 à 20 mg/kg, par voie intrapéritonéale ou par voie orale.

Les résultats des essais effectués sur les composés de l'invention montrent qu'ils sont des inhibiteurs des transporteurs de la glycine glyt1 présents dans le cerveau et de la glycine glyt2 présents dans le cerveau ou la moelle épinière.

Les composés selon l'invention peuvent donc être utilisés pour la préparation de médicaments, en particulier de médicaments inhibiteurs des transporteurs de la glycine glyt1 et/ou glyt2.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Les composés de l'invention peuvent être utilisés notamment des troubles comportementaux associés à la démence, des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.

Par ailleurs, les composés de l'invention peuvent être utilisés pour le traitement des contractures musculaires douloureuses en rhumatologie et en pathologie rachidienne aiguë, pour le traitement des contractures spastiques d'origine médullaire ou cérébrale, pour le traitement symptomatique des douleurs aiguës et subaiguës d'intensité légère à modérée, pour le traitement des douleurs intenses et/ou chroniques, des douleurs neurogènes et algies rebelles, pour le traitement de la maladie de Parkinson et des symptômes parkinsoniens d'origine neurodégénérative ou induits par des neuroleptiques, pour le traitement des épilepsies généralisées primaires et secondaires, partielles à symptomatologie simple ou complexe, des formes mixtes et autres syndromes épileptiques en complément d'un autre traitement antiépileptique, ou en monothérapie, pour le traitement des apnées du sommeil, et pour la neuroprotection.

La présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec un ou plusieurs excipients convenables. Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également l'utilisation d'un composé selon l'invention pour la préparation d'un médicament destiné au traitement des pathologies ci-dessus indiquées, ledit traitement comprenant l'administration, à un patient, d'une dose efficace d'un composé selon l'invention, ou un de ses sels pharmaceutiquement acceptables ou hydrates ou solvats.

## Revendications

1. Composé répondant à la formule générale (I) 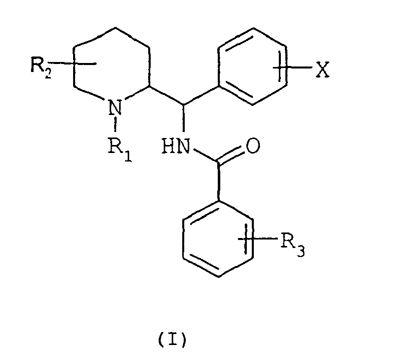 dans laquelle
R₁ représente soit un atome d'hydrogène, soit un groupe (C₁-C₇) alkyle linéaire ou ramifié éventuellement substitué par un ou plusieurs atomes de fluor, soit un groupe (C₃-C₇)-cycloalkyle, soit un groupe (C₃-C₇) cycloalkyl (C₁-C₃) alkyle, soit un groupe phényl(C₁-C₃)alkyle éventuellement substitué par un ou deux groupes méthoxy, soit un groupe (C₂-C₄)alcényle, soit un groupe (C₂-C₄) alcynyle ;
R₂ représente soit un groupe (C₁-C₇) alkyle linéaire ou ramifié ou (C₃-C₇) cycloalhyle, soit un groupe (C₃-C₇) cycloalkyl (C₁-C₃) alkyle;
X représente soit un atome d'hydrogène, soit un ou plusieurs substituants choisis parmi les atomes d'halogène et les groupes trifluorométhyle, (C₁-C₆)alkyle linéaire ou ramifié ou (C₁-C₆)alcoxy;
R₃ représente soit un atome d'hydrogène, soit un ou plusieurs substituants choisis parmi les atomes d'halogène et les groupes trifluorométhyle, (C₁-C₆)alkyle linéaire ou ramifié, (C₃-C₇)cycloalkyle, (C₁-C₆) alcoxy, phényle, cyano, acétyle, benzoyle, (C₁-C₆) thioalkyle, (C₁-C₆)alkylsulfonyle, carboxy ou (C₁-C₆) alcoxycarbonyl, soit un groupe de formule générale NR₄R₅ ou SO₂NR₄R₅ ou CONR₄R₅ dans lesquelles R₄ et R₅ représentent chacun, indépendamment l'un de l'autre, un atome d'hydrogène ou un groupe (C₁-C₆)alkyle linéaire ou ramifié ou (C₃-C₇)cycloalkyle, ou R₄ et R₅ forment, avec l'atome d'azote qui les porte, un cycle pyrrolidine, pipéridine ou morpholine ; à l'état de base libre ou de sel d'addition à un acide, d'hydrate ou de solvat.

2. Composé selon la revendication 1, **caractérisé en ce qu'**il est de configuration thréo ; à l'état de base libre ou de sel d'addition à un acide, d'hydrate ou de solvat.

3. Composé selon la revendication 2, **caractérisé en ce qu'**il est de configuration (1*S*, 2*S*) ou (1*R*, 2*R*) ; à l'état de base libre ou de sel d'addition à un acide, d'hydrate ou de solvat.

4. Composé selon la revendication 1, **caractérisé en ce qu'**il est de configuration érythro; à l'état de base libre ou de sel d'addition à un acide, d'hydrate ou de solvat.

5. Composé selon la revendication 4, **caractérisé en ce qu'**il est de configuration (1*S*, 2*R*) ou (1*R*, 2*S*); à l'état de base libre ou de sel d'addition à un acide, d'hydrate ou de solvat.

6. Médicament **caractérisé en ce qu'**il comprend un composé selon l'une des revendications 1 à 5, ou un sel d'addition de ce composé à un acide pharmaceutiquement acceptable ou encore un hydrate ou un solvat du composé de formule (I).

7. Composition pharmaceutique, **caractérisée en ce qu'**elle comprend un composé selon l'une des revendications 1 à 5, ou un sel pharmaceutiquement acceptable, un hydrate ou un solvat de ce composé, ainsi qu'au moins un excipient pharmaceutiquement acceptable.

8. Utilisation d'un composé de formule (I selon l'une des revendications 1 à 5 pour la préparation d'un médicament destiné au traitement des troubles comportementaux associés à la démence, des psychoses, des diverses formes d'anxiété, des attaques de panique, des phobies, des troubles obsessionnels compulsifs, des différentes formes de dépression, des troubles dus à l'abus ou au sevrage d'alcool, des troubles du comportement sexuel, des troubles de la prise de nourriture et de la migraine.

9. Utilisation d'un composé de formule (I) selon l'une des revendications 1 à 5 pour la préparation d'un médicament destiné au traitement des contractures, de la douleur, de la maladie de Parkinson et des symptômes parkinsoniens, des épilepsies, des formes mixtes et autres syndromes épileptiques en complément d'un autre traitement antiépileptique, ou en monothérapie, des apnées du sommeil, et pour la neuroprotection.

## Claims

1. Compound corresponding to the general formula (I) 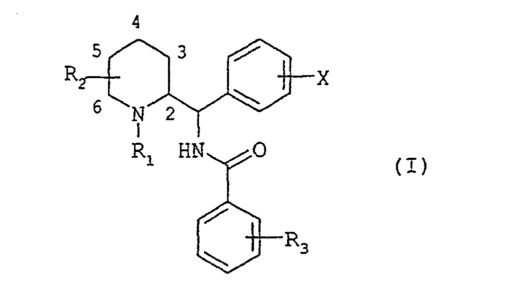 in which
R₁ represents either a hydrogen atom, or a linear or branched (C₁-C₇) alkyl group optionally substituted with one or more fluorine atoms, or a (C₃-C₇) cycloalkyl group, or a (C₃-C₇) cycloalkyl (C₁-C₃)alkyl group, or a phenyl(C₁-C₃)alkyl group optionally substituted with one or two methoxy groups, or a (C₂-C₄)alkenyl group, or a (C₂-C₄)alkynyl group;
R₂ represents either a linear or branched (C₁-C₇)alkyl or (C₃-C₇)cycloalkyl group, or a (C₃-C₇)cycloalkyl (C₁-C₃)alkyl group;
X represents either a hydrogen atom or one or more substituents chosen from halogen atoms and trifluoromethyl, linear or branched (C₁-C₆) alkyl and (C₁-C₆) alkoxy groups;
R₃ represents either a hydrogen atom or one or more substituents chosen from halogen atoms and trifluoromethyl, linear or branched (C₁-C₆) alkyl, (C₃-C₇)cycloalkyl, (C₁-C₆) alkoxy, phenyl, cyano, acetyl, benzoyl, (C₁-C₆)thioalkyl, (C₁-C₆) alkylsulfonyl, carboxyl and (C₁-C₆) alkoxycarbonyl groups, or a group of general formula NR₄R₅ or SO₂NR₄R₅ or CONR₄R₅ in which R₄ and R₅ each independently represent a hydrogen atom or a linear or branched (C₁-C₆) alkyl or (C₃-C₇)cycloalkyl group, or R₄ and R₅ form, with the nitrogen atom that bears them, a pyrrolidine, piperidine or morpholine ring; in the form of free base or acid-addition salt, hydrate or solvate.

2. Compound according to Claim 1, **characterized in that** it is of threo configuration; in the form of free base or acid-addition salt, hydrate or solvate.

3. Compound according to Claim 2, **characterized in that** it is of (1*S*, 2*S*) or (1*R*, 2*R*) configuration; in the form of free base or acid-addition salt, hydrate or solvate.

4. Compound according to Claim 1, **characterized in that** it is of erythro configuration; in the form of free base or acid-addition salt, hydrate or solvate.

5. Compound according to Claim 4, **characterized in that** it is of (1*S*, 2*R*) or (1*R*, 2*S*) configuration; in the form of free base or acid-addition salt, hydrate or solvate.

6. Medicament, **characterized in that** it comprises a compound according to one of Claims 1 to 5, or an addition salt of this compound with a pharmaceutically acceptable acid or alternatively a hydrate or a solvate of the compound of formula (I).

7. Pharmaceutical composition, **characterized in that** it comprises a compound according to one of Claims 1 to 5, or a pharmaceutically acceptable salt, a hydrate or a solvate of this compound, and also at least one pharmaceutically acceptable excipient.

8. Use of a compound of formula (I) according to one of Claims 1 to 5, for the preparation of a medicament for treating behavioral disorders associated with dementia, psychoses, various forms of anxiety, panic attacks, phobias, obsessive compulsive disorders, various forms of depression, disorders caused by alcohol abuse or weaning from alcohol, sexual behavior disorders, eating disorders and migraine.

9. Use of a compound of formula (I) according to one of Claims 1 to 5 for the preparation of a medicament for treating contracture, pain, Parkinson's disease and Parkinson-like symptoms, epilepsy, mixed forms and other epileptic syndromes in addition to another antiepileptic treatment, or in monotherapy, and sleep apnea, and for neuroprotection.

## Patentansprüche

1. Verbindung der allgemeinen Formel (I) 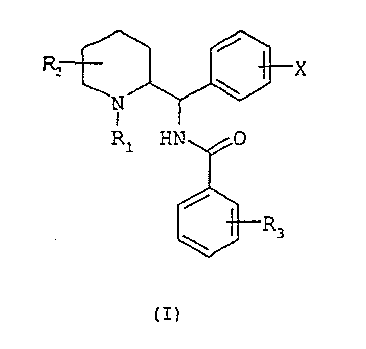 worin
R₁ entweder für ein Wasserstoffatom oder eine lineare oder verzweigte C₁-C₇-Alkylgruppe steht, die eventuell durch ein oder mehrere Fluoratome oder eine C₃-C₇-Cycloalkylgruppe oder eine C₃-C₇-Cycloalkyl-C₁-C₃-alkylgruppe oder eine C₁-C₃-Alkylphenylgruppe substituiert ist, die eventuell durch eine oder zwei Methoxygruppen oder eine C₂-C₄-Alkenylgruppe oder eine C₂-C₄-Alkinylgruppe substituiert ist;
R₂ entweder für eine lineare oder verzweigte oder C₃-C₇-Cycloalkyl-C₁-C₇-alkylgruppe oder für eine C₃-C₇-Cycloalkyl-C₁-C₃-alkylgruppe steht;
X entweder für ein Wasserstoffatom oder einen oder mehrere Substituenten steht, ausgewählt aus den Halogenatomen und Trifluormethyl-, linearen oder verzweigten C₁-C₆-Alkyl- oder C₁-C₆-Alkoxygrüppen;
R₃ entweder für ein Wasserstoffatom oder einen oder mehrere Substituenten steht, ausgewählt aus den Halogenatomen und Trifluormethyl-, linearen oder verzweigten C₁-C₆-Alkyl-, C₃-C₇-Cycloalkyl-, C₁-C₆-Alkoxy-, Phenyl-, Cyano-, Acetyl-, Benzoyl-, C₁-C₆-Thioalkyl-, C₁-C₆-Alkylsulfonyl-, Carboxy- oder C₁-C₆-Alkoxycarbonylgruppen oder für eine Gruppe der allgemeinen Formel NR₄R₅ oder SO₂NR₄R₅ oder CONR₄R₅ worin R₄ und R₅ jeweils unabhängig voneinander für ein Wasserstoffatom oder eine lineare oder verzweigte oder C₃-C₇-Cycloalkyl-C₁-C₆-alkylgruppe stehen, oder R₄ und R₅ mit dem Stickstoffatom, das sie trägt, einen Pyrrolidin-, Piperidin- oder Morpholinring bilden; im Zustand einer freien Base oder eines Säureadditionssalzes, eines Hydrats oder eines Solvats.

2. Verfahren nach Anspruch 1, **dadurch gekennzeichnet, dass** es eine Threo-Konfiguration ist; im Zustand einer freien Base oder eines Säureadditionssalzes, eines Hydrats oder eines Solvats.

3. Verfahren nach Anspruch 2, **dadurch gekennzeichnet, dass** es eine (1*S*, 2*S*)- oder (1*R*, 2*R*)-Konfiguration ist; im Zustand einer freien Base oder eines Säureadditionssalzes, eines Hydrats oder eines Solvats.

4. Verfahren nach Anspruch 1, **dadurch gekennzeichnet, dass** es eine Erythro-Konfiguration ist; im Zustand einer freien Base oder eines Säureadditionssalzes, eines Hydrats oder eines Solvats.

5. Verfahren nach Anspruch 4, **dadurch gekennzeichnet, dass** es eine (1*S*, 2*R*)- oder (1*R*, 2*S*) -Konfiguration ist; im Zustand einer freien Base oder eines Säureadditionssalzes, eines Hydrats oder eines Solvats.

6. Medikament, **dadurch gekennzeichnet, dass** es eine Verbindung nach einem der Ansprüche 1 bis 5 oder ein Additionssalz dieser Verbindung mit einer pharmazeutisch unbedenklichen Säure oder auch ein Hydrat oder ein Solvat der Verbindung der Formel (I) umfasst.

7. Pharmazeutische Zusammensetzung, **dadurch gekennzeichnet, dass** sie eine Verbindung nach einem der Ansprüche 1 bis 5 oder ein pharmazeutisch unbedenkliches Salz, ein Hydrat oder ein Solvat dieser Verbindung sowie mindestens einen pharmazeutisch unbedenklichen Hilfsstoff umfasst.

8. Verwendung einer Verbindung der Formel (I) nach einem der Ansprüche 1 bis 5 zur Herstellung eines Medikaments, das zur Behandlung von Verhaltensstörungen, die mit Demenz verbunden sind, Psychosen, diversen Angstformen, Panikattacken, Phobien, Zwangsstörungen, verschiedenen Depressionsformen, Störungen, die auf Alkoholmissbrauch oder Alkoholentzug zurückzuführen sind, Störungen des Sexualverhaltens, Störungen der Nahrungsaufnahme und der Migräne, bestimmt ist.

9. Verwendung einer Verbindung der Formel (I) gemäß einem der Ansprüche 1 bis 5 zur Herstellung eines Medikaments, das zur Behandlung von Kontrakturen, Schmerz, Parkinsonkrankheit und Parkinsonähnlichen Symptomen, Epilepsien, Mischformen und anderen epileptischen Syndromen als Ergänzung einer anderen antiepileptischen Behandlung oder als Monotherapie von Schlafapnoe und zur Neuroprotektion bestimmt ist.
